(19) Europäisches Patentamt
European Patent Office
Office européen des brevets

(11) Publication number : 0 577 580 A2

(12) # EUROPEAN PATENT APPLICATION

(21) Application number : 93870087.9

(22) Date of filing : 26.05.93

(51) Int. Cl.[5] : C12P 19/26

(30) Priority : 26.05.92 US 888939

(43) Date of publication of application :
05.01.94 Bulletin 94/01

(84) Designated Contracting States :
AT BE CH DE DK ES FR GB GR IE IT LI LU MC NL PT SE

(71) Applicant : MONSANTO COMPANY
800 North Lindbergh Boulevard
St. Louis Missouri 63167 (US)

(71) Applicant : NEW ENGLAND MEDICAL CENTER HOSPITALS, INC.
750 Washington Street, Box 817
Boston Massachusetts 02111 (US)

(72) Inventor : Jacob, Gary Steven
12541 Mason Forest
Creve Coeur, Missouri 63141 (US)
Inventor : Scudder, Peter Richard
1940 Dovershire Court
Chesterfield, Missouri 63017 (US)
Inventor : Welply, Joseph Kevin
5 Edgecrest Court
St. Peters, Missouri 63376 (US)
Inventor : Pereira, Miercio Expedito Alves
478 Clinton Road
Chestnut Hill, Massachusetts 02167 (US)

(74) Representative : Ernst, Hubert et al
Monsanto Services International S.A., Patent Department, Avenue de Tervuren 270-272, Letter Box No. 21
B-1150 Brussels (BE)

(54) **Synthesis of sialoconjugates.**

(57) There is disclosed a method of synthesizing α2,3-sialylated conjugates using trans-sialidase.

EP 0 577 580 A2

## BACKGROUND OF THE INVENTION

### Field of the Invention

This invention pertains to enzymatic synthesis and more particularly to a method of synthesizing α2,3-sialylated conjugates using trans-sialidase.

### Description of the related art

Oligosaccharide sequences of glycoconjugates which contain sialic acid are involved in a diverse array of biological functions. Noteable is their role in facilitating certain critical cell-cell interactions (e.g., in embryogenesis, growth, differentiation and oncogenesis) and their ability to function as receptors for pathogens, proteins, and toxins, Paulson, *The Receptors* Vol. 2, 131-219, Academic Press, New York [1985], Loomes et al., *Nature* 307, 560-563 [1984], Schauer, *Adv. Carbohydr. Chem. Biochem.* 40, 131-234 [1982], Hakomori, *Annu. Rev. Immunol.* 2, 103-126 [1984]. Recently, structures based on sialylated Lewis$^x$ (NeuAcα2-3Galβ1-4GlcNAc-[Fucα1-3]GlcNAc) and sialylated Lewis$^a$ (NeuAcα2-3Galβ1-3GlcNAc[Fucα1-4]GlcNAc) antigens have been shown to mediate binding of circulating leukocytes to vascular endothelial cells. Tyrell et al., *Proc. Nat. Acad. Sci.* USA 88, 10372-10376 [1991]. This event, a component of the acute inflammatory response, occurs through an interaction between the sialylated sugars on the leukocyte and a glycoprotein receptor, termed E-selectin, on the endothelial cell.

These and other biologically active oligosaccharides are difficult to purify from natural sources in amounts sufficient for biochemical study. Chemical methods for the synthesis of complex sugars have advanced rapidly over recent years but suffer from low yields (especially for large-scale work), primarily due to the need to isolate each intermediate after the many blocking and deblocking steps that are involved, e.g. synthesis of the sialyl-Lewis$^x$ tetrasaccharide shown above requires approximately 24 steps. Not surprisingly there is an increasing interest in the use of enzymes to synthesize oligosaccharides. Two main classes of enzyme have been utilized, glycosyltransferases and glycosidases.

Glycosyltransferases (EC 2.4) catalyze the transfer of a monosaccharide from a nucleotide sugar (CMP-NeuAc, UDP-Gal, GDP-Fuc etc) to the non-reducing terminus of an appropriate saccharide acceptor as shown below:

Nucleotide-Sugar$_1$ + Sugar$_2$ ---------> Nucleotide + Sugar$_1$-Sugar$_2$

These enzymes exhibit absolute regio- stereospecificity with respect to the linkage that is formed and can generate high yields of product. However, their use for large-scale synthesis is often compromised by their limited abundence and by the expense of the nucleotide sugar donor-substrate which is involved in the biotransformation.

Glycosidases (EC 3.2) which normally catalyze the hydrolysis of glycosidic linkages (see Equation 1) can, under appropriate conditions (Nilsson, *Trends Biotechnol.* 6, 256-264 [1988] WO89/09275), be utilized for the synthesis of oligosaccharides in kinetically-controlled, or transglycosylation, reactions (see Equation 2):

### Equation 1

Monosaccharide-X + $H_2O$ ---> Monosaccharide + X

### Equation 2

Monosaccharide-X + Y ---> Monosaccharide-Y + X

where X and Y can be mono- or oligosaccharides.

However, in general glyosidases are very inefficient at catalyzing the transglycosylation reaction (giving low product yields) and demonstrate poor regioselectivity (which results in a mixture of enzyme-products).

It has been recognized for more than a decade that trypomastigotes of *Trypanosoma cruzi* express a developmentally regulated neuraminidase-like activity which has been implicated in the parasite invasion of cells (Prioli et al., *J. Immunol.* 144, 4384-4391 [1990]). Recently, Schenkman et al. (*Cell* 65, 1117-1125 [1991]) showed that, *in vivo,* this glycosidase demonstrates trans-sialidase activity and can catalyze the transfer of sialic acid from host cell sialoglycoconjugates to a parasite-specific oligosaccharide epitope which is involved in the adhesion of *Trypanosoma cruzi* to target cells. An oligosaccharide product, generated *in vitro* by this enzyme utilizing lactose (Galβ1-4Glc) as an acceptor-substrate, was not characterized structurally but, by thin layer chromatography, co-chromatographed with authentic 2,3-sialyllactose, SAα2-3Galβ1-4Glc. Similar results have also been obtained by Parodi et. al., (*EMBO J.* 11, 1705-1710 [1992]). The mode of action of this

enzyme thus appears to differ fundamentally from glycosyltransferases and glycosidases.

## SUMMARY OF THE INVENTION

In accordance with the present invention a novel process for synthesizing oligosaccharides is provided of the formula SAα2-3Galβ1-$R_2$, which comprises:

reacting a trans-Sialidase on SAα2-R, as a donor in the presence of Galβ1-$R_2$ as the acceptor;

wherein $R_1$ and $R_2$ are independently selected from the group consisting of saccharide, glycolipid, glycoprotein, glycopeptide, acyclic or aliphatic hydrocarbons or aryl residue; and

SA is any naturally occuring sialic acid or derivative thereof.

It is an object of the present invention to utilize trans-sialidase in an efficient and predictable manner for the large-scale synthesis of a2,3-sialylated glycoconjugates.

## BRIEF DESCRIPTION OF THE DRAWINGS

Fig. 1 shows trans-sialidase-catalyzed release of sialic acid in the presence and absence of an acceptor-substrate.

Fig. 2 shows the effect of donor-substrate (2,3-sialyllactose, 2,3-SL) concentration on the synthesis of 2,3-sialyllactosamine.

Fig. 3 thin layer chromatogram of trans-sialidase reaction products utilizing the glycolipid, paragloboside, as an acceptor.

## DETAILED DESCRIPTION OF THE INVENTION

Trans-sialidase is identified as an enzyme that will be of extreme value for the large-scale synthesis of a broad variety of sialylated conjugates including oligosaccharides, glycoproteins, glycopeptides, glycolipids and carbohydrate mimetics. Two important examples of oligosaccharides that have been synthesized include NeuAcα2-3Galβ1-3GlcNAcβ1-3Galβ1-4Glc and NeuAcα2-3Galβ1-4GlcNAcβ1-3Galβ1-4Glc, precursors of sialyl-Lewis$^a$ and sialyl-Lewis$^x$, respectively. The trans-sialidase isolated from *Trypanosoma cruzi* is shown to be a unique enzyme which specifically catalyzes the synthesis of sialoglycans according to the following general equation:

SAα2-$R_1$ + Galb1-$R_2$ -------> SAa2-3Galb1-$R_2$ + $R_1$

where $R_1$ and $R_2$ can be a saccharide, glycolipid, glycoprotein, glycopeptide, acyclic or cyclic aliphatic group, or aryl residue and SA can be any naturally-occurring sialic acid, modified form of sialic acid or sialic acid analogue. The donor-substrate for this reaction is preferably NeuAcα2-3Galβ1-4G1c (2,3-sialyllactose) or 2-O-(p-nitrophenyl)-N-acetyl-α-D-neuraminic acid. The product terminates in an α2-3 sialosidic linkage. Retention of the α-anomeric configuration indicates that the mechanism of action of this enzyme differs significantly from sialyl- and other glycosyltransferases which generate a linkage having anomeric inversion. Synthesis using trans-sialidase occurs without significant hydrolysis of either the sialylated substrate or product as monitored by the release of free sialic acid. An efficient method whereby trans-sialidase can be immobilized on Concanavalin-A with retention of catalytic activity has been developed. The activity of the parasite-derived enzyme is such that it can be utilized, either free in solution or in an immobilized form, for the efficient large-scale synthesis of sialoglycans which terminate in the sequence SAα2-3Ga1b1-$R_2$.

Trans-sialidase was isolated from trypomastigotes of *Trypanosoma cruzi.* Epimastigotes were cultured in a liver-infusion medium containing 10% fetal calf serum and trypomastigotes were propogated in Vero cells as previously described the following references are hereby incorporated by reference (Libby et al. *J. Clin. Invest.* 77, 127-135 [1986], Pereira and Hoff, *Mol. Biochem. Parasitol.* 20, 183-189 [1986]). Trypomastigotes were then incubated in culture-medium minus fetal calf serum and including protease inhibitors (Libby et al. *J. Clin. Invest.* 77, 127-135 [1986]) for 72 h at 37°C and the parasites pelleted by centrifugation. The spontaneously released enzyme present in the supernatant was stored in the presence of 50% glycerol at -20°C until required. Prior to use, glycerol was removed by desalting the enzyme on a Quick Spin™ protein column (Boehringer Manheim) prewashed with 25 mM cacodylate/HCl buffer, pH 6.8, containing 2 mg/ml BSA (buffer A). The enzyme was routinely assayed by incubation with 10 μm $^{14}C$-lactose (55 Ci/mol) and 2 mM 2,3-sialyllactose (purified from sialyllactose mixed isomers obtained from Sigma) in buffer A. The concentration of lactose and 2,3-sialyllactose was significantly below the $K_m$ for each substrate. The $^{14}C$-labeled sialylated product was recovered by S-Sepharose anion exchange chromatography and was measured by scintillation counting. The activity of the partially-purified enzyme was typically of the order of 0.2 milliunits/ml of culture medium (20 units/mg protein). When assayed against saturating concentrations (10 mM) of 2,3-sialyllactose and N-acetyllactosamine, Galβ1-4GlcNAc (an alternative acceptor-substrate) the activity of the trans-sialidase was 0.2 units/ml (formation of the product was monitored by triple-pulsed amperometry following separation of the reaction products by high performance anion exchange chromatography using a Dionex BioLc System). The product of sialic acid transfer to N-acetyllactosamine was isolated by S-Sepharose anion-exchange chromatography and shown by methylation analysis which is hereby incorporated by reference (Scudder et al. *Eur. J. Biochem.* 168, 585-593 [1987]) to be SAα2-3Galβ1-4GlcNAc. Trans-sialidase demonstrated activity towards a number of oligosaccharides terminating in β-, but not α-, linked galactose. Each of the sialylated products was shown by methylation analysis to contain a terminal sialic acid linked α2-3 with galactose. A summary of these results is given in Table 1. Trans-sialidase cannot utilize oligosaccharides which terminate in NeuAcα2-6Gal as donor-substrates. The trans-sialidase reaction is not significantly compromised by hydrolytic activity since the enzyme demonstrates extremely low levels of neuraminidase activity both in the presence and absence of an acceptor-substrate (see Fig. 1). The sialylation reaction can be driven to near completion by utilizing an excess of the donor-substrate, i.e., 2,3-sialyllactose, over the acceptor-substrate (see Fig. 2). The utility of the trans-sialidase was enhanced by prior immobilization of the enzyme using lectin affinity chromatography as follows: The enzyme preparation was stirred for 30 min at 4°C with Concanavalin-A-Sepharose (20% by vol.) in the presence of 1 mM $CaCl_2$ and the gel was washed with 20 volumes of buffer A containing 1 mM $CaCl_2$. In this form the enzyme was reutilized without detectable loss of activity.

Trans-sialidase-catalyzed release of sialic acid in the presence and absence of an acceptor-substrate is shown in Fig 1. Two 100 μl reaction mixtures were set up at 37°C. One contained 3.4 μunits of trans-sialidase, 50 mM cacodylate/HCl buffer, pH 6.9, 50 nmol of 2,3-sialyllactose and 0.2 mg of BSA. The second was identical but contained, in addition, 50 nmol of N-acetyllactosamine (Galβ1-4GlcNAc) as an acceptor-substrate. High peformance anion exchange chromatography using pulsed amperometric detection was used to monitor the rate of trans-sialidase-catalyzed synthesis of 2,3-sialyllactosamine (o) and the rate of sialic released in the presence (o) and absence (●) of N-acetyllactosamine.

Effect of donor-substrate (2,3-sialyllactose, 2,3-SL) concentration on the synthesis of 2,3-sialyllactosamine is shown in Fig. 2.

Trans-sialidase, 1.6 μunits, was incubated at 37°C in 50 μl of 50 mM cacodylate/HCl buffer, pH 6.9, containing 50 nmol of N-acetyllactosamine, Galβ1-4GlcNAc, 0.1 mg of BSA, and different amounts of 2,3-sialyllactose (0.05, 0.25, 1.0 or 2.0 nmol). Aliquots were removed at various time intervals and the amount of 2,3-sialyllactosamine formed was determined by high peformance anion exchange chromatography using pulsed amperometric detection.

A preferred embodiment of the present invention is the following equation

SAα2-3Galβ1-$R_1$ + Galβ1-$R_2$ -------> SAα2-3Galβ1-$R_2$ + Galβ1-$R_1$

Wherein $R_1$ or $R_2$ is as defined above. $R_1$ is more preferably any alkyl or aryl residue. $R_2$ is more preferably a saccharide residue.

In order to further illustrate the invention, the following exemplary laboratory preparative work was carried out. However, it will be appreciated that the invention is not limited to these examples or the details described therein.

## EXAMPLE 1

Synthesis of NeuAcα2-3Galβ1-3GlcNAcβ1-3Galβ1-4Glc Using Concanavalin A-Immobilized Trans-Sialidase.

0.4 ml of Concanavalin A-Sepharose-trans-sialidase (40 microunits/ml as assayed against $^{14}$C-lactose) was added to 0.9 ml of 50 mM cacodylate/HCl buffer, pH 6.9, containing 20 μmol of Galβ1-3GlcNAcβ1-3Galβ1-4Glc, 40 μmol of NeuAcα2-3Galβ1-4Glc, 2 mg of bovine serum albumin and 0.02% sodium azide. The suspension was mixed by inversion for 16 h at 37°C. The gel was removed by filtration and washed with buffer A containing 1 mM $CaCl_2$. The sialylated product was purified by chromatography on a column of TSK-HW40(S) equilibrated with 50 mM acetic acid and lyophilized to give 16 μmol (16.1 mg) of pure (assessed by $H^1$-NMR and methylation analysis) NeuAcα2-3Galβ1-3GlcNAcβ1-3Galβ1-4Glc. As an example of the significance of this synthesis we have been able to fucosylate the sialylated pentasaccharide product using an immobilized form of human milk α3/4-fucosyltransferase to generate the sialyl-Lewis$^a$ structure NeuAcα2-3Galβ1-3(Fucα1-4)GlcNAcβ1-3Galβ1-4(Fucα1-3)Glc. This reaction utilized a donor-substrate of GDP-fucose.

## Example 2

Synthesis of NeuAcα2-3Galβ1-4GlcNAcβ1-3Galβ1-4Glc Synthesis was as described in Example 1 but utilizing 16 μunits (as assayed against $^{14}$C-lactose) of soluble enzyme and 20 μmol of Galβ1-4GlcNAcβ1-3Galβ1-4Glc as the acceptor-substrate.

**Example 3**

Synthesis of the Sialylated Glycoprotein, NeuAcα2-3Galβ1-4Glc-BSA The neoglycoprotein, Galβ1-4Glc-BSA (1 mg, 23.2 mol Galβ1-4Glc/mol BSA) was dissolved in 1 ml of 50 mM cacodylate/HCl buffer, pH 6.8, containing 12 μmol of 2,3-sialyllactose, 16 μunits of trans-sialidase and 0.02% sodium azide. After incubation at 37°C for 16 h the sialylated glycoprotein was recovered by gel filtration on a column of TSK-HW40(S) equilibrated in 50 mM ammonium bicarbonate and lyophilized. The product, which had a sialic acid content of 22 mol/mol of BSA, was completely susceptible to the action of Newcastle Disease virus neuraminidase indicating that the structure of the synthesized oligosaccharide linked to BSA was NeuAcα2-3Galβ1-4Glc.

**Example 4**

Synthesis of the Sialylated Glycosphingolipid, NeuAcα2-3Galβ1-4GlcNAcβ1-3Galβ1-4Glcβ1-Ceramide. Paragloboside (Galβ1-4GlcNAcβ1-3Galβ1-4Glcβ1-ceramide), 1.25 nmol, was dissolved in 6.25 μl of 0.1M cacodylate/HCl buffer, pH 6.9, containing 0.1% Triton X-100, 0.5 mg/ml BSA, 1.25 nmol 2,3-sialyllactose and 3.6 μunits of trans-sialidase and incubated at 22°C for 3 h. HP-TLC (See Fig. 3) showed the formation of a glycolipid product which corresponded to NeuAcα2-3Galβ1-4GlcNAcβ1-3Galβ1-4Glcβ1-Ceramide. . Susceptibility of the sialylated glycolipid to hydrolysis by Newcastle Disease virus neuraminidase confirmed that the sialosidic linkage formed was NeuAcα2-3Gal.

Thin layer chromatogram of trans-sialidase reaction products utilizing the glycolipid, paragloboside, as an acceptor is shown in Fig. 3. Lane 1, standards including sialylparagloboside (SPG); Lane 2, standards including paragloboside (PG); Lane 3, reaction mixture at zero time; Lane 4, reaction mixture at 3 h.; Lane 5, 2,3-sialyllactose; Lane 6, lactose.

From the foregoing description, one skilled in the art can easily ascertain the essential characteristics of this invention, and without departing from the spirit and scope thereof, can make various changes and modifications of the invention to adapt it to various usages and conditions.

**Table 1**

| Acceptor-Substrate | Relative Rate |
|---|---|
| Galβ1-4GlcNAcβ1-3Galβ1-4Glc | 100 |
| Galβ1-4GlcNAc | 58 |
| Galβ1-3GlcNAcβ1-3Galβ1-4Glc | 35 |
| Galβ1-3GlcNAc | 4 |
| Galβ1-3GlcNAcβ1-3Galβ1-4Glc \|α1,3 Fuc | 35 |
| Galβ1-6GlcNAc | 45 |
| Galβ1-4Galβ1-4Glc | 41 |
| Galβ1-4Man | 25 |
| Galβ1-3GalNAc | 35 |
| Galβ1-6Gal | 30 |
| Galβ1-4gluconic acid | 55 |

**Claims**

1. A process for synthesizing oligosaccharides of the Formula SAα2-3Galβ1-$R_2$, which comprises:
reacting a trans-Sialidase on SAα2-$R_1$ as a donor in the presence of Galβ1-$R_2$ as the receptor;
wherein $R_1$ and $R_2$ are independently selected from the group consisting of saccharide, glycolipid, glycoprotein, glycopeptide, acyclic or aliphatic hydrocarbons or aryl residue; and

SA is any naturally occuring sialic acid or derivative thereof.

**2.** The process as recited in claim 1 wherein said donor is SA$\alpha$2-3Gal$\beta$1-$R_1$.

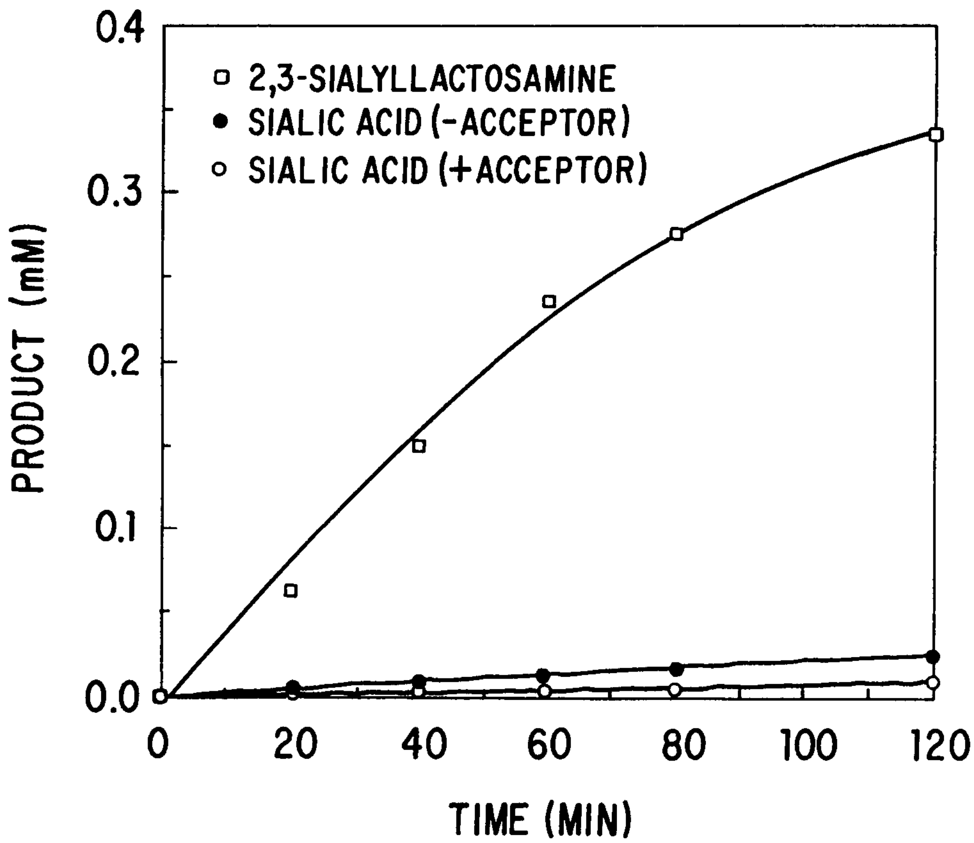
0.4
0.3
0.2
0.1
0.0
PRODUCT (mM)
2,3-SIALYLLACTOSAMINE
SIALIC ACID (-ACCEPTOR)
SIALIC ACID (+ACCEPTOR)
0
20
40
60
80
100
120
TIME (MIN)

FIG. 1

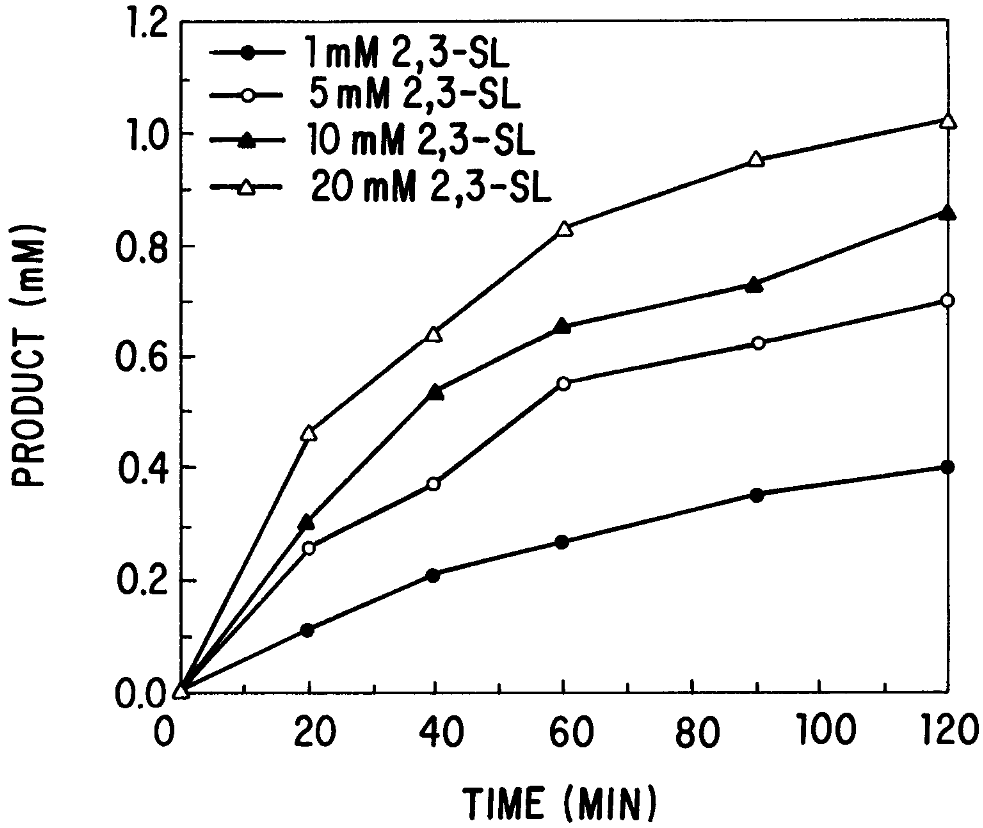
1.2
1.0
0.8
0.6
0.4
0.2
0.0
PRODUCT (mM)
1 mM 2,3-SL
5 mM 2,3-SL
10 mM 2,3-SL
20 mM 2,3-SL
0
20
40
60
80
100
120
TIME (MIN)

FIG. 2

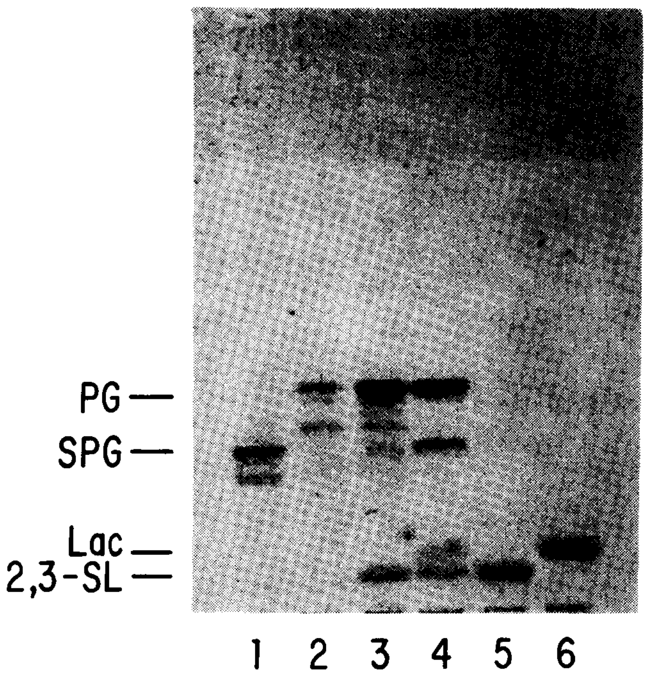


FIG. 3